# EUROPEAN PATENT SPECIFICATION

(11) **EP 3 192 518 B1**
(45) Date of publication and mention of the grant of the patent: **20.01.2021**
(21) Application number: 15839889.1
(22) Date of filing: 09.09.2015
(51) Int. Cl.: A61K 35/20, A61P 29/00, A61P 43/00

(54) **ANTI-INFLAMMATORY AGENT**
ENTZÜNDUNGSHEMMENDER WIRKSTOFF
AGENT ANTI-INFLAMMATOIRE

(30) Priority: 09.09.2014 JP 2014183061
(43) Date of publication of application: 19.07.2017
(73) Proprietor: Morinaga Milk Industry Co., Ltd., Minato-ku Tokyo 108-8384 (JP)
(72) Inventor: IZUMI, Hirohisa, Zama-shi Kanagawa 252-8583 (JP); TSUDA, Muneya, Zama-shi Kanagawa 252-8583 (JP)
(74) Representative: Vossius & Partner Patentanwälte Rechtsanwälte mbB
(86) International application number: PCT/JP2015/075529
(87) International publication number: WO 2016/039356

(56) References cited:
- EP-A1- 2 687 219
- WO-A1-2010/065652
- WO-A1-2014/134132
- TAKETOSHI HATA ET AL: "Isolation of bovine milk-derived microvesicles carrying mRNAs and microRNAs", BIOCHEMICAL AND BIOPHYSICAL RESEARCH COMMUNICATIONS, vol. 396, no. 2, 1 May 2010 (2010-05-01), pages 528-533, XP055113144, ISSN: 0006-291X, DOI: 10.1016/j.bbrc.2010.04.135
- ONNO J. ARNTZ ET AL: "Oral administration of bovine milk derived extracellular vesicles attenuates arthritis in two mouse models", MOLECULAR NUTRITION & FOOD RESEARCH, vol. 59, no. 9, 1 July 2015 (2015-07-01), pages 1701-1712, XP055446691, DE ISSN: 1613-4125, DOI: 10.1002/mnfr.201500222
- HIROHISA IZUMI ET AL: "Bovine milk exosomes contain microRNA and mRNA and are taken up by human macrophages", JOURNAL OF DAIRY SCIENCE., vol. 98, no. 5, 1 May 2015 (2015-05-01), pages 2920-2933, XP055446704, US ISSN: 0022-0302, DOI: 10.3168/jds.2014-9076
- ADMYRE, C. ET AL.: 'Exosomes with immune modulatory features are present in human breast milk' JOURNAL OF IMMUNOLOGY vol. 179, no. 3, 2007, pages 1969 - 1978, XP055113095
- AQIL, F. ET AL.: 'Milk derived exosomes: Scalable source of biologically active drug delivery nanoparticles' AACR ANNUAL MEETING 2014 PRESENTATION ABSTRACT 09 April 2014, XP009500966 Retrieved from the Internet: <URL:http://www.abstractsonline.com/Plan/Vi ew Abstract.aspx?sKey=7d16a559-b063-4a63-a20d- 0a3404da849d&cKey=3dc2e526-5047-4bc9-bla4- 9831a409de0e&mKey={6FFE1446-A164-476A-92E7- C26446874D93> [retrieved on 2015-10-23]
- HIROHISA IZUMI ET AL.: 'Boshikan o Ido suru Bonyuchu Exosome no Igi' EXPERIMENTAL MEDICINE vol. 29, no. 3, 2011, pages 399 - 403, XP008171074
- KOSUKE MURAKAMI ET AL.: 'Ushi Nyusei kara Chosei shita Makushoho Kakubun ni Sonzai suru Tanpakushitsu no Moratei Kaiseki' JAPAN SOCIETY FOR BIOSCIENCE, BIOTECHNOLOGY, AND AGROCHEMISTRY TAIKAI KOEN YOSHISHU 2005, page 96, XP009500997
- AZIZ, M. ET AL.: 'Treatment of Recombinant Mouse MFG-E8 Downregulates LPS-Induced TNF-alpha Production in Macrophages via STAT3-Mediated SOCS3 Activation' PLOS ONE vol. 6, no. 11, 2011, page E27685, XP055417154 Retrieved from the Internet: <URL:http://journals.plos.org/plosone/artic le?id=10. 1371/journal.pone.0027685> [retrieved on 2015-10-23]

## Description

### Technical Field

The present invention relates to an anti-inflammatory agent comprising a milk-derived ingredient as an active ingredient.

### Background Art

Anti-inflammatory agents include steroidal anti-inflammatory agents and non-steroidal anti-inflammatory agents. Although steroidal anti-inflammatory agents exhibit outstanding anti-inflammatory activity, they also cause many side reactions including immunosuppressive action. As also for non-steroidal anti-inflammatory agents, such side reactions as alimentary canal ulcer, various allergic responses, and renal disorder are known.

As a food-derived anti-inflammatory agent expected to be highly safe, there is known an anti-inflammatory agent comprising an extract of red ginger (*Zingiber officinale* var. *rubrum*) as an active ingredient (Patent document 1). This extract contains anthocyanidin and gingerol.

By the way, it is known that exosomes, which are membrane vesicles secreted from biological cells or cultured cells, exist in body fluids such as blood (serum and plasma), mother's milk, alveolar lavage, malignant pleural effusion fluid, synovia, urine, amniotic fluid, sperm, saliva, and so forth. Exosomes contain messenger RNA (mRNA), microRNA (miRNA), double stranded DNA, protein, lipid, and so forth (Non-patent documents 1 and 2). It is known that composition of these RNAs and proteins differs depending on type of cell that secretes exosomes, and so forth (Non-patent documents 3 and 4), and it is thought that activity and function of exosomes differ depending on the origin thereof.

It has been reported that exosomes prepared from culture of antigen presenting cells such as dendritic cells and macrophages collected from tissues of bone marrow, spleen, lymph node, thymus, etc. or peripheral blood inhibit immunological response (Patent document 2).

Exosomes or exosome-like vesicles also exist in plants, and it has been reported that the exosome-like nanoparticles of vine induce intestinal stem cells, and prevent dextran sulfate sodium (DSS)-induced colitis of mice (Non-patent document 5).

It has also been reported that a pharmaceutical composition comprising corticosteroid and exosomes in combination can be used for treatment of articular diseases such as arthritis (Patent document 3).

As for milk, it has been reported that when membrane vesicles were prepared from milk (colostrum or mature milk), and added to the Raw 264 .7 cells, which are rodential macrophage type cells, and cytokines contained in the supernatant were evaluated, although amount of the tumor necrosis factor α did not change, IL-1β, IL-6, which are inflammatory cytokines similarly to tumor necrosis factor α, and so forth increased, and IL-10, which is an anti-inflammatory cytokine, decreased (Non-patent document 6), and it has been suggested that membrane vesicles contained in milk promote inflammation.

It is also becoming clear that immunosuppressive actions that function in an inhibitory manner on allergic responses, autoimmune diseases, chronic inflammation, and so forth are controlled by miRNA. It was found that miRNA also exists in milk, and it is known that milk containing increased amount of such miRNA has a potent immunostimulating action (Patent document 4). It is further reported that this miRNA exists in exosomes (Non-patent document 7).

It is also known that by increasing amount of a specific mRNA contained in milk, the immunostimulating action of the milk can be increased (Patent document 5).

miRNA and mRNA contained in exosomes of milk were further analyzed, and it has been reported that exosomes are taken up by macrophages (Non-patent document 8). It has also been reported that surface antigens of exosomes contained in milk differ depending on whether allergy sensitization is performed or not, or lifestyle (Non-patent document 9).

However, it was unknown that exosomes contained in milk have a tumor necrosis factor α production-suppressing action or anti-inflammatory action, and the action is not provided by RNA contained in exosomes. Specifically, none of the prior art documents disclosed or proposed the use of a disrupted exosome in the therapy of inflammation.

### Prior art references

### Patent documents

Patent document 1: Japanese Patent No. 5303697
Patent document 2: International Patent Publication WO2006/007529
Patent document 3: International Patent Publication WO2011/082950
Patent document 4: U.S. Patent Published Application No. 20120093874
Patent document 5: U.S. Patent Published Application No. 20130280368
Patent document 6: WO2014/134132 A1
Patent document 7: EP 2 687 219 A1
Patent document 8: WO2010/065652 A1

### Non-patent documents

Non-patent document 1: Saadeldin I.M. et al. Stem Cells and Cloning: Advances and Applications, 8:103-107, 2015
Non-patent document 2: Ramachandran S. et al., Wiley Interdiscip. Rev. RNA, 3:286-293, 2012
Non-patent document 3: Douglas D. Taylor and Cicek Gercel-Taylor, Frontiers in Genetics, 4(142):1-12, 2013
Non-patent document 4: Simpson, R.J. et al., Expert Rev. Proteomics, 6, 267-283, 2009
Non-patent document 5: Songwen, Ju, et. al., Molecular Therapy, 21(7): 1345-1357, 2013
Non-patent document 6: Sun, Q., et al., Protein Cell, 4(3):197-210, 2013
Non-patent document 7: Kosaka, N., et al., Silence 1:7, 2010
Non-patent document 8: Izumi H. et al., J. Dairy Sci., 98:2920-2933, 2015
Non-patent document 9: Paredes et al., Allergy, 69:463-471, 2014
Non-patent document 10: Hata, T. et al., Biochemical and Biophysical Research Communications 396: 528-533, 2010.
Non-patent document 11: Arntz, O. et al., Molecular Nutrition & Food Research 59(9): 1701-1712, 2015.
Non-patent document 12: Admyre, C. et al., Journal of Immunology 179(3): 1969-1978, 2007.
Non-patent document 13: Aqil, F. et al., AACR Annual Meeting 2014 Presentation abstract, 2014.
Non-patent document 14: Izumi, H. et al., Experimental Medicine 29(3): 399-403, 2011.
Non-patent document 15: Murakami, K. et al., Japan Society for Bioscience, Biotechnology and Agrochemistry Taikai Koen Yoshishu: 96, 2005.
Non-patent document 16: Aziz, M. et al., Plos One 6(11): E27685, 2011.

### Summary of the Invention

### Object to be Achieved by the Invention

An object of the present invention is to provide a highly safe anti-inflammatory agent.

### Means for Achieving the Object

The inventors of the present invention found that exosomes derived from milk have a tumor necrosis factor α production-suppressing action, and are suitable as an ingredient of an anti-inflammatory agent, and accomplished the present invention.

The present invention thus provides a milk-derived disrupted exosome the membrane vesicle of which is partially or completely disrupted for use in a therapeutic treatment or prophylactic treatment of inflammation or an inflammatory disease.

The present invention also provides a milk-derived disrupted exosome the membrane vesicle of which is partially or completely disrupted for use as a drug for anti-inflammation. According to a preferred embodiment, the milk-derived disrupted exosome for use in the present invention has a tumor necrosis factor α production-suppressing action.

The present invention also provides a method for producing an anti-inflammatory agent, which comprises:
the step of removing lipid fraction, cell fraction and casein fraction from milk to prepare a whey fraction,
the step of collecting an exosome fraction from the whey fraction, and
the step of disrupting membranes of exosomes by ultrasonication,
and
the step of making the disrupted exosome fraction as an active ingredient into a preparation.

### Brief Description of the Drawings

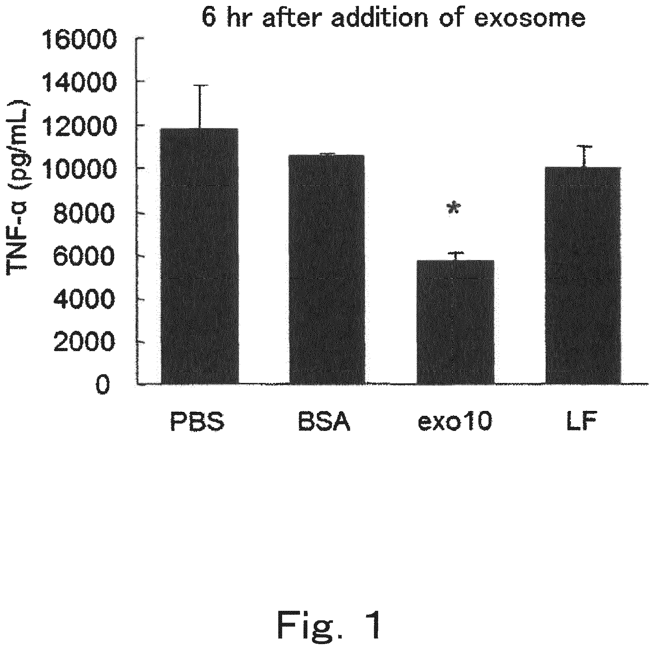

Fig. 1 shows a graph showing the tumor necrosis factor α production-suppressing action of milk-derived exosomes against macrophage-like cells: BSA stands for bovine serum albumin, exo10 for exosome fraction, and LF for bovine lactoferrin. The symbol "*" means that there is significance at p < 0.05 in the Dunnett test, in which PBS group was used as control.

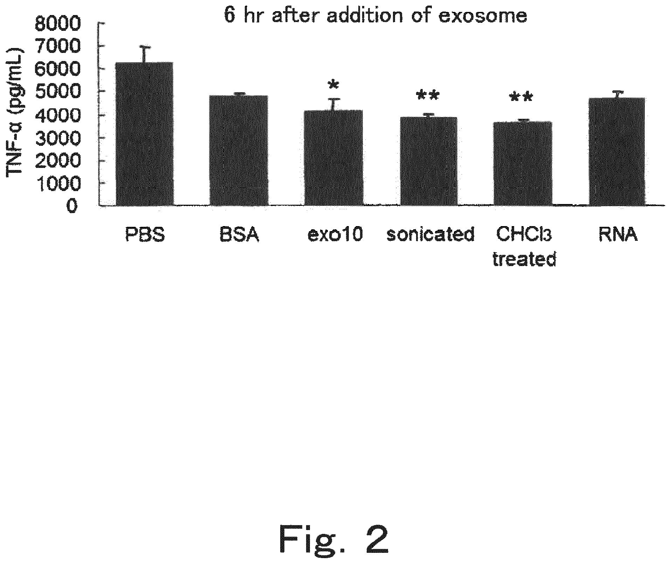

Fig. 2 shows a graph showing the tumor necrosis factor α production-suppressing action of milk-derived exosomes against macrophage-like cells: BSA stands for bovine serum albumin, exo10 for exosome fraction, sonicated for the exosome fraction subjected to ultrasonication, CHCl₃ treated for the exosome fraction treated with chloroform, and RNA for RNA purified from the exosome fraction. The symbols "*" and "**" mean that there are significances at p < 0.05 and P < 0.01, respectively, in the Dunnett test, in which PBS group was used as control.

### Embodiments for Carrying out the Invention

Hereafter, preferred embodiments of the present invention will be explained in detail. However, the present invention is not limited to the following embodiments, but can be freely changed within the scope of the present invention.

The anti-inflammatory agent of the present invention comprises a milk-derived disrupted exosome an an active ingredient. That is, the exosome used for the present invention is an exosome obtained from milk. The milk is not particularly limited so long as it is milk obtained from a mammal, and examples include milk obtained from human, cow, goat, sheep, pig, monkey, dog, cat, rat, mouse, hamster, guinea pig, or the like. Preferred examples include milk obtained from human or cow. Although the milk may be colostrum or mature milk, mature milk is preferred.

The milk-derived exosome is an exosome contained in milk, and it is synonymous with exosome obtained from milk. The exosome, which is a kind of membrane vesicle, is a microparticle covered with a lipid bilayer membrane, and containing lipids, proteins, RNAs (mRNA, miRNA) and DNAs. The diameter thereof is said to be 20 to 120 nm, or 30 to 100 nm (Saadeldin I.M. et al., Stem Cells and Cloning: Advances and Applications, 8:103-107, 2015). As described later, the milk-derived disrupted exosome as the active ingredient of the anti-inflammatory agent of the invention is an exosome of which membrane vesicle structure has been disrupted. Therefore, the "milk-derived exosome" contained in the anti-inflammatory agent is a microparticle whose lipid bilayer has been disrupted.

Although the method for preparing the exosome from milk is not particularly limited, an example is described below as a specific method.

Lipid fraction, cell fraction, and casein fraction are removed from milk to prepare a whey (milk serum) fraction. An exosome fraction is collected from the obtained whey fraction. The exosome fraction can be collected by using a known method such as ultracentrifugation method, filtration method, density gradient centrifugation method, sucrose cushion method, ultrafiltration method, continuous flow electrophoresis method, and chromatography method (International Patent Publication WO2014/030590). Specifically, if the whey fraction is centrifuged at 30,000 to 1,000,000 × g, for example, 100,000 × g, for 60 to 120 minutes, and the supernatant is removed, an exosome fraction is obtained.

Preparation of exosomes from whey is described in published references, for example, Izumi H. et al., J. Dairy Sci., 98:2920-2933, 2015.

Kits for isolating exosomes from biological fluids such as serum, plasma, urine, and cerebrospinal fluid are also marketed (for example, Exosome Isolation Kit from Takara Bio, and Exosome Isolation Reagent from Life Technology), and they may also be used.

Exosome fraction means a fraction having an exosome content higher than those of the other fractions obtained by fractionating raw material milk. In the present invention, exosomes in the exosome fraction are preferably concentrated to a content 3 times or more, preferably 5 times or more, more preferably 10 times or more, further preferably 25 times or more, higher than that of the raw material milk. The content of exosomes can be represented as, for example, content (mass %) based on the total solid amount. The content of exosomes can also be measured by counting number of exosomes in a sample using a nanoparticle analyzer (for example, NanoSight, Quantum Design Japan), or the like. Alternatively, the exosome fraction preferably contains a protein specifically contained in exosomes at a ratio relative to the total protein content higher by 3 times or more, preferably 5 times or more, more preferably 10 times or more, further preferably 25 times or more, compared with that of milk. Examples of the protein specifically contained in exosomes include, for example, CD63. The exosome fraction may be one consisting of substantially only exosomes, solution thereof, or suspension thereof. Amount or concentration of protein can be measured by, for example, the Lowry method, Bradford method, or an improved method of these methods. Amount or concentration of a specific protein can be measured by enzyme immunoassay, Western blotting, or the like.

By making the obtained exosome fraction as the active ingredient into a preparation, an anti-inflammatory agent is obtained. The milk-derived exosome has an action for suppressing production of tumor necrosis factor α (henceforth referred to as "TNF-α"), which is a kind of inflammatory cytokine, and a composition containing milk-derived exosomes may also be a TNF-α production-suppressing agent.

The exosome used for the present invention is a disrupted exosome of which membrane vesicle structure is partially or completely disrupted. As shown in the examples, at least the anti-inflammatory activity or TNF-α production-suppressing action of the milk-derived exosome (henceforth also referred to simply as "exosome") is not provided by RNA contained in the exosomes alone, and it is highly possible that an ingredient other than RNA contributes to the activity. Therefore, the exosome used for the present invention may contain RNA, or may not contain RNA. Examples of the method for removing RNA from an exosome include, for example, an appropriate fractionation method using organic solvent treatment, ultrasonic disruption, or the like.

When the anti-inflammatory agent of the present invention is pharmaceutically formulated, additives such as excipient, binder, disintegrating agent, lubricant, stabilizer, corrigant, diluent, and solvent for injections can be used. Specific examples of pharmaceutical preparation include tablet (including sugar-coated tablet, enteric coated tablet, and buccal tablet), powder, capsule (including enteric capsule and soft capsule), granule (including coated granule), pill, troche, liposome-enclosed agent, solution, and pharmaceutically acceptable sustained release preparations of these. However, milk, whey, and compositions containing any of them not subjected to a process for increasing exosome content are excluded from the anti-inflammatory agent of the present invention.

Examples of carrier and excipient used for such preparations as mentioned above include lactose, glucose, sucrose, mannitol, potato starch, corn starch, calcium carbonate, calcium phosphate, calcium sulfate, crystalline cellulose, glycyrrhizae radix pulverata, gentianae radix pulverata, and so forth, and examples of binder include starch, gelatin, syrup, polyvinyl alcohol, polyvinyl ether, polyvinylpyrrolidone, hydroxypropylcellulose, ethylcellulose, methylcellulose, carboxymethylcellulose, and so forth.

Examples of disintegrating agent include starch, agar, gelatin powder, sodium carboxymethylcellulose, calcium carboxymethylcellulose, crystalline cellulose, calcium carbonate, sodium hydrogencarbonate, sodium arginate, and so forth.

Examples of lubricant include magnesium stearate, hydrogenated vegetable oil, macrogol, and so forth, and examples of colorant include red No. 2, yellow No. 4, blue No. 1, of which addition to pharmaceuticals is allowed, and so forth.

Tablets and granules may be coated as required with sucrose, hydroxypropylcellulose, purified shellac, gelatin, sorbitol, glycerin, ethyl cellulose, hydroxypropylcellulose, hydroxypropylmethylcellulose, polyvinylpyrrolidone, cellulose acetate phthalate, hydroxypropylmethylcellulose phthalate, methyl methacrylate, methacrylic acid polymer, or the like.

The object of application of the anti-inflammatory agent of the present invention is not particularly limited so long as it is inflammation, especially inflammation in which TNF-α is involved, or a disease accompanied by inflammation, i.e. an inflammatory disease, and examples include, for example, metabolic syndromes such as obesity; enterocolitis, allergy, and so forth. By administering the anti-inflammatory agent of the present invention, these inflammations and diseases can be treated or prevented. However, the anti-inflammatory agent of the present invention does not include a pharmaceutical composition comprising corticosteroid together with exosomes, and to be used for treatment of an articular disease.

One aspect of the present invention is a method for producing an anti-inflammatory agent, which comprises the step of removing lipid fraction, cell fraction and casein fraction from milk to prepare a whey fraction, the step of collecting an exosome fraction from the whey fraction, the step of disrupting membranes of exosomes by ultrasonication and the step of making the disrupted exosome fraction as an active ingredient into a preparation. Another aspect of the present invention is a milk-derived disrupted exosome for use as a drug for anti-inflammation. A further aspect of the present invention is a milk-derived disrupted exosome for use in a therapeutic treatment or prophylactic treatment of inflammation or a disease accompanied by inflammation, i.e. an inflammatory disease. A still further aspect of the present disclosure is a method for a therapeutic treatment or prophylactic treatment of inflammation or a disease accompanied by inflammation, which comprises administering a milk-derived exosome or an anti-inflammatory agent comprising a milk-derived exosome to an animal or human. Examples of the inflammation or inflammatory disease include inflammation or inflammatory disease in which TNF-α is involved, and examples of the animal or human include an animal or human having such a disease or disease accompanied by inflammation as mentioned above.

Although the anti-inflammatory agent of the present invention may be administered by oral administration or parenteral administration, oral administration is preferred. Examples of the parenteral administration include intravenous injection, rectal administration, inhalation, and so forth. Examples of dosage form for oral administration include tablet, capsule, troche, syrup, granule, powder, ointment, and so forth. A known drug or pharmaceutical composition having an anti-inflammatory activity, or such a drug or pharmaceutical composition to be found in future can also be used together. The pharmaceutical composition used together may be contained in the agent of the present invention as one of the active ingredients, or may not be contained in the agent of the present invention, but combined as a separate agent with the agent of the present invention to constitute a commercial product.

By making the milk-derived exosome as an active ingredient to be contained in a food, a food having anti-inflammatory activity may also be prepared as an embodiment of the anti-inflammatory agent.

Form of such a food as mentioned above is not particularly limited, and it may be in the form of liquid, paste, solid, powder, or the like. Examples include tablet confectioneries, liquid diets, feeds (including feed for pets), and so forth, as well as, for example, flour products such as bread, macaroni, spaghetti, noodles, cake mix, fry powder and bread crumbs; ready-to-eat foods such as instant noodles, pot noodles, retort and cooked foods, canned cooking, foods for microwave heating, instant soup and stew, instant miso soup and Japanese clear soup, canned soup, freeze-dried foods, and other ready-to-eat foods; processed agricultural products such as canned agricultural products, canned fruits, jams and marmalades, pickles, cooked beans, dry agricultural products, and cereals (processed grain products); processed marine products such as canned marine products, fish ham and sausages, seafood paste products, marine dainties, and tsukudani (marine products boiled in soy source); processed livestock products such as canned livestock products and pastes, and livestock meat ham and sausages; milks and dairy products such as processed milk, milk drinks, yoghurts, lactic fermenting beverages, cheese, ice creams, modified milk powders, creams, and other dairy products; oils and fats such as butter, margarines, and vegetable oils; basic seasoning such as soy sauce, miso, sauces, processed tomato seasoning, mirin, and vinegars; complex seasonings and foods such as cooking mix, curry powder or roux, sauces for dipping, dressings, noodle soups, spices, and other complex seasonings; frozen foods such as frozen food materials, semi-cooked frozen foods, and cooked frozen foods; confectioneries such as caramel candies, candies, chewing gums, chocolates, cookies, biscuits, cakes, pies, snacks, crackers, Japanese sweets, rice confectioneries, bean confectioneries, dessert pastries, and other confectioneries; beverages such as carbonated drinks, natural fruit juices, fruit juice drinks, fruit juice soft drinks, fruit pulp drinks, fruit drinks with fruit pulp, vegetable based drinks, soy milk, soy milk drinks, coffee drinks, tea drinks, powdered drinks, concentrated drinks, sports drinks, nutritional beverage, alcoholic drinks, and other beverages; other commercial foods such as baby foods, rice seasonings, and seaweed seasonings for boiled rice soaked with tea; modified milk powder for infants; enteral nutrients; functional foods (foods for specified health use, foods with nutrient function claims), and so forth.

Furthermore, by adding the milk-derived exosome as an active ingredient to a feed, a feed having an anti-inflammatory activity can be prepared, as one embodiment of the anti-inflammatory agent.

Form of the feed is not particularly limited. For example, the feed can be prepared by blending milk-derived exosomes with cereals such as corn, wheat, barley, rye and milo; vegetable oil meals such as soybean oil meal, rapeseed oil meal, coconut oil meal and linseed oil meal; brans such as wheat bran, rice bran, and defatted rice bran; production meals such as corn gluten meal and corn jam meal; animal or fish-derived feeds such as fish meal, skim milk powder, whey, yellow grease and tallow; yeasts such as torula yeast and brewer' s yeast; mineral material feeds such as tribasic calcium phosphate and calcium carbonate; oils and fats; monomeric amino acids; saccharides, and so forth. Examples of the form of the feed include, for example, pet food, livestock feed, fish breeding feed, and so forth.

In the anti-inflammatory agent of the present invention (including the embodiments of drug, food or drink, feed, quasi drug, and so forth), formulation amount of the exosome is preferably 0.01 mass % or more, particularly preferably 0.1 mass % or more, based on the mass of the final composition of the anti-inflammatory agent.

Although dose of the anti-inflammatory agent of the present invention varies depending on age, condition, etc., amount of the exosomes is usually 1 mg to 10 g per day, preferably 100 mg to 1 g per day, in terms of protein amount, and the agent of such a dose may be administered once a day or several times a day as divided portions. If the anti-inflammatory agent of the present invention is taken or ingested, the effect of the present invention is fully exhibited irrespective of time of taking or ingestion of the agent, for example, between meals, or before or after meal.

### Examples

Hereafter, the present invention will be more specifically explained with reference to examples. However, the present invention is not limited to these examples.

### (1) Preparation of milk-derived exosomes

An operation of centrifuging raw material milk used for the manufacture of dairy products at 1,200 × g and 4°C for 10 minutes to remove fats and cells contained in the raw material milk was repeated twice to obtain skim milk. 10% Acetic acid was added dropwise to the obtained skim milk to reduce pH to 4.6, and thereby precipitate casein. The skim milk at pH 4.6 was centrifuged at 4,500 × g and 4°C for 30 minutes to remove casein. Then, an operation of centrifuging the skim milk at 20,000 × g and 4°C for 30 minutes, and collecting supernatant was performed twice to completely remove casein. The obtained sample was filtered through filters (0.45 µm and 0.22 µm, Millipore) to obtain milk serum (whey) . The obtained milk serum was ultracentrifuged at 100,000 × g and 4°C for 90 minutes, the precipitates in the form of pellet containing exosomes were suspended in PBS, and the suspension was ultracentrifuged in the same manner as described above to wash the exosomes. This washing was performed again to obtain an exosome fraction. From 1 ml of the raw material milk, 10 µg (in terms of protein amount) of the exosome fraction was obtained.

### (2) Confirmation of anti-inflammatory activity (I)

Cells of the human monocytic leukemia cell strain, THP-1 (ATCC TIB-202), were cultured in a medium (complete medium) containing 10% inactivated fetal bovine serum (HyClone, exosomes contained in the serum were removed by ultracentrifugation), 1% penicillin/streptomycin/glutamine solution (Gibco), 1% nonessential amino acid mixture (Gibco), and 0.1% β-mercaptoethanol, then 50 nM PMA (phorbol 12-myristate 13-acetate, Sigma) was added to the medium, and the culture was continued for 72 hours so that the cells differentiated into macrophage-like cells. A cell suspension containing 5 × 10⁴ of the cells in 80 µl of the complete medium, and each of test substance solutions containing one of the following test substances (ii) to (iv) dissolved in 20 µl of PBS (solution of (i) consisted of 20 µl of PBS alone) were mixed to obtain test solutions (100 µl). The amounts of the test substances (ii) to (iv) corresponded to the amounts of the test substances contained in 20 µl, 1 ml, and 100 µl of milk, or 10 µl, 500 µl, and 50 µl of whey, respectively.
(i) PBS
(ii) Bovine serum albumin (BSA) in an amount of 10 µg
(iii) Exosome fraction (exo10) in an amount of 10 µg in terms of protein amount
(iv) Bovine lactoferrin (LF) in an amount of 10 µg

Each prepared test solution was incubated at 37°C for 6 hours under a 5% CO₂ environment, then the culture supernatant was removed, and the cells were washed twice with PBS. After the washing, 100 µl of the complete medium was added to the cells, LPS (lipopolysaccharide, Sigma) was added to the cell suspension at 100 ng/mL, and culture was performed for 16 hours. The culture supernatant was collected after 16 hours, and amount of the inflammatory cytokine, TNF-α, contained in the supernatant was measured by using an immunoassay kit (Milliplex, Millipore), and evaluated.

The results are shown in Fig. 1. As a result, the TNF-α production amount observed for the control (PBS) was 11834 pg/ml, and TNF-α production amounts obtained with bovine serum albumin and lactoferrin were both about 10000 pg/ml. In contrast, the TNF-α production amount obtained with the exosomes was 5738 pg/ml, and it was confirmed that they suppressed the TNF-α production amount even to about 1/2 of that observed for the control. It became clear that the milk-derived exosomes suppressed the production of TNF-α by the macrophage-like cells, and they have an anti-inflammatory activity.

### (3) Confirmation of anti-inflammatory activity (II)

The membranes of the exosomes were disrupted by ultrasonication or organic solvent treatment, and it was investigated whether the form of membrane vesicle would be required for the anti-inflammatory activity of the exosomes. Further, since it was confirmed in "Confirmation of anti-inflammatory activity (I)" that albumin and lactoferrin, which are proteins contained in the exosomes, added to the cells in an amount equivalent to that of the exosomes do not show anti-inflammatory activity, it was then decided to examine action of RNA contained in the exosomes by extracting RNA from the exosomes by the acid guanidine-phenol-chloroform method, and adding RNA purified by using miRNeasy Serum/Plasma Kit (Qiagen) to the cells. A cell suspension containing 5 × 10⁴ of the THP-1 cells, which were differentiated into macrophage-like cells in the same manner as described above, in 80 µl of the complete medium, and each of test substance solutions containing one of the following test substances (ii) to (vi) dissolved in 20 µl of PBS (solution of (i) consisted of 20 µl of PBS alone) were mixed to obtain test solutions (100 µl).
(i) PBS
(ii) Bovine serum albumin in an amount of 10 µg
(iii) Exosome fraction in an amount of 10 µg in terms of protein amount
(iv) Exosome fraction in an amount of 10 µg in terms of protein amount, which was treated by ultrasonication^{*1}
(v) Exosome fraction in an amount of 10 µg in terms of protein amount, which was treated with chloroform^{*2}
(vi) RNA purified from the exosome fraction in an amount of 10 µg in terms of protein amount
   *1: The exosome fraction was ultrasonicated 5 times for 20 seconds for each time by using an ultrasonic disruptor US-300E produced by Nissei.
   *2: Chloroform and methanol were added to the exosome solution at a ratio of exosome solution:chloroform:methanol = 1:1.25:2.5, the mixture was stirred and then centrifuged to remove the organic solvent, and the exosomes were re-suspended in ultrapure water.

Each prepared test solution was incubated at 37°C for 6 hours under a 5% CO₂ environment, then the culture supernatant was removed, and the cells were washed twice with PBS. After the washing, 100 µl of the complete medium was added to the cells, and LPS (lipopolysaccharide, Sigma) was added to the cell suspension at 100 ng/mL. The culture supernatant was collected after 16 hours, and amount of the inflammatory cytokine, TNF-α (Tumor Necrosis Factor α), contained in the supernatant was measured by using an immunoassay kit (Milliplex, Millipore), and evaluated.

The results are shown in Fig. 2. Even when the form of membrane vesicle of the milk-derived exosomes was disrupted by ultrasonication or chloroform treatment, the ultrasonicated exosomes provided a TNF-α production amount of 3736 pg/ml, and the chloroform-treated exosomes provided a TNF-α production amount of 3567 pg/ml, and thus they showed suppression of TNF-α production equivalent to or higher than that obtainable with the intact exosomes (exo10, TNF-α production amount was 4081 pg/ml). RNA extracted from the exosomes did not show significant suppression of the production of TNF-α (TNF-α production amount: 4597 pg/ml) . On the basis of these results, it was confirmed that the anti-inflammatory activity of the milk-derived exosomes does not require the membrane structure, and the anti-inflammatory activity is not provided by RNA alone, and it was suggested that an ingredient other than RNA contributes to the activity.

### Industrial Applicability

The anti-inflammatory agent of the present invention has a tumor necrosis factor α (TNF-α) production-suppressing action, and is useful as an anti-inflammatory agent. The raw material of the anti-inflammatory agent of the present invention is milk that is widely used as food or drink, and it is thought that it is highly safe as food or drink, drug, and so forth.

## Claims

1. A milk-derived disrupted exosome the membrane vesicle of which is partially or completely disrupted for use in a therapeutic treatment or prophylactic treatment of inflammation or an inflammatory disease.

2. A milk-derived disrupted exosome the membrane vesicle of which is partially or completely disrupted for use as a drug for anti-inflammation.

3. The milk-derived disrupted exosome for use according to claim 1 or 2, wherein the inflammation is inflammation involving tumor necrosis factor α.

4. The milk-derived disrupted exosome for use according to claim 1, wherein the inflammatory disease is an inflammatory disease involving tumor necrosis factor α.

5. The milk-derived disrupted exosome for use according to claim 1 or 4, wherein the inflammatory disease is an inflammatory disease selected from metabolic syndromes, enterocolitis and allergy.

6. The milk-derived disrupted exosome for use according to any one of claims 1 to 5, which has a tumor necrosis factor α production-suppressing action.

7. A method for producing an anti-inflammatory agent, which comprises:
the step of removing lipid fraction, cell fraction and casein fraction from milk to prepare a whey fraction,
the step of collecting an exosome fraction from the whey fraction,
the step of disrupting membranes of exosomes by ultrasonication, and
the step of making the disrupted exosome fraction as an active ingredient into a preparation.

8. The milk-derived disrupted exosome for use according to any one of claims 1 to 6, wherein the milk-derived disrupted exosome is prepared by a method comprising:
the step of removing lipid fraction, cell fraction and casein fraction from milk to prepare a whey fraction,
the step of collecting an exosome fraction from the whey fraction and
the step of disrupting membranes of exosomes.

9. The milk-derived disrupted exosome for use according to claim 8,
wherein ultrasonication or organic solvent treatment is used in the step of disrupting membranes of exosomes.

## Patentansprüche

1. Aus Milch stammendes aufgeschlossenes Exosom, dessen Membranvesikel teilweise oder vollständig aufgeschlossen ist, zur Verwendung bei einer therapeutischen Behandlung oder prophylaktischer Behandlung einer Entzündung oder einer entzündlichen Erkrankung.

2. Aus Milch stammendes aufgeschlossenes Exosom, dessen Membranvesikel teilweise oder vollständig aufgeschlossen ist, zur Verwendung als entzündungshemmendes Medikament.

3. Aus Milch stammendes aufgeschlossenes Exosom zur Verwendung nach Anspruch 1 oder 2, wobei die Entzündung eine Entzündung ist, an der der Tumornekrosefaktor α beteiligt ist.

4. Aus Milch stammendes aufgeschlossenes Exosom zur Verwendung nach Anspruch 1, wobei die entzündliche Erkrankung eine entzündliche Erkrankung ist, an der der Tumornekrosefaktor α beteiligt ist.

5. Aus Milch stammendes aufgeschlossenes Exosom zur Verwendung nach Anspruch 1 oder 4, wobei die entzündliche Erkrankung eine entzündliche Erkrankung ist, die ausgewählt ist aus metabolischen Syndromen, Enterocolitis und Allergie.

6. Aus Milch stammendes aufgeschlossenes Exosom zur Verwendung nach einem der Ansprüche 1 bis 5, das eine die Tumornekrosefaktor α-Herstellung unterdrückende Wirkung aufweist.

7. Verfahren zur Herstellung eines entzündungshemmenden Agens, das umfasst:
den Schritt des Entfernens der Lipidfraktion, Zellfraktion und Caseinfraktion aus der Milch, um eine Molkefraktion herzustellen,
den Schritt des Gewinnens einer Exosomfraktion aus der Molkefraktion,
den Schritt des Aufschließens von Membranen von Exosomen durch Ultraschall, und
den Schritt des Herstellens eines Präparats aus dem aufgeschlossenen Exosom als Wirkstoff.

8. Aus Milch stammendes aufgeschlossenes Exosom zur Verwendung nach einem der Ansprüche 1 bis 6, wobei das aus Milch stammende Exosom hergestellt wird durch ein Verfahren, das umfasst:
den Schritt des Entfernens der Lipidfraktion, Zellfraktion und Caseinfraktion aus der Milch, um eine Molkefraktion herzustellen,
den Schritt des Gewinnens einer Exosomfraktion aus der Molkefraktion, und
den Schritt des Aufschließens von Membranen von Exosomen.

9. Aus Milch stammendes aufgeschlossenes Exosom zur Verwendung nach Anspruch 8, wobei eine Behandlung mit Ultraschall oder einem organischen Lösungsmittel im Schritt des Aufschließens der Membranen von Exosomen verwendet wird.

## Revendications

1. Exosome fragmenté dérivé du lait dont la vésicule membranaire est partiellement ou totalement fragmentée, destiné à être utilisé dans le cadre d'un traitement thérapeutique ou d'un traitement prophylactique d'une inflammation ou d'une maladie inflammatoire.

2. Exosome fragmenté dérivé du lait dont la vésicule membranaire est partiellement ou totalement fragmentée, destiné à être utilisé comme un médicament anti-inflammatoire.

3. Exosome fragmenté dérivé du lait destiné à être utilisé selon la revendication 1 ou 2, dans lequel l'inflammation est une inflammation qui implique un facteur de nécrose tumorale α.

4. Exosome fragmenté dérivé du lait destiné à être utilisé selon la revendication 1, dans lequel la maladie inflammatoire est une maladie inflammatoire qui implique un facteur de nécrose tumorale α.

5. Exosome fragmenté dérivé du lait destiné à être utilisé selon la revendication 1 ou 4, dans lequel la maladie inflammatoire est une maladie inflammatoire choisie parmi des syndromes métaboliques, une entérocolite et une allergie.

6. Exosome fragmenté dérivé du lait destiné à être utilisé selon l'une quelconque des revendications 1 à 5, qui possède une action de suppression de production de facteur de nécrose tumorale α.

7. Procédé de fabrication d'un agent anti-inflammatoire, qui comprend :
l'étape de suppression de la fraction lipidique, de la fraction cellulaire et de la fraction de caséine du lait afin de préparer une fraction de lactosérum,
l'étape de collecte d'une fraction d'exosome à partir de la fraction de lactosérum,
l'étape de fragmentation des membranes d'exosomes par ultrasonication, et
l'étape de constitution de la fraction d'exosome fragmentée comme un ingrédient actif dans une préparation.

8. Exosome fragmenté dérivé du lait destiné à être utilisé selon l'une quelconque des revendications 1 à 6, dans lequel l'exosome fragmenté dérivé du lait est préparé par un procédé comprenant :
l'étape de suppression de la fraction lipidique, de la fraction cellulaire et de la fraction de caséine du lait afin de préparer une fraction de lactosérum,
l'étape de collecte d'une fraction d'exosome à partir de la fraction de lactosérum, et
l'étape de fragmentation des membranes d'exosomes.

9. Exosome fragmenté dérivé du lait destiné à être utilisé selon la revendication 8,
dans lequel une ultrasonication ou un traitement par solvant organique est utilisé(e) à l'étape de fragmentation des membranes d'exosomes.
